# EUROPEAN PATENT SPECIFICATION

(11) **EP 4 321 115 B1**
(45) Date of publication and mention of the grant of the patent: **03.06.2026**
(21) Application number: 23175146.2
(22) Date of filing: 24.05.2023
(51) Int. Cl.: A61B 17/34, A61B 90/96, A61B 17/15, A61B 17/17, A61B 17/00, A61B 34/10, A61B 34/20, A61B 90/00

(54) **SYSTEM FOR USER GUIDANCE IN SURGICAL NAVIGATION**
SYSTEM ZUR BENUTZERFÜHRUNG IN DER CHIRURGISCHEN NAVIGATION
SYSTÈME DE GUIDAGE D'UTILISATEUR DANS UNE NAVIGATION CHIRURGICALE

(30) Priority: 11.08.2022 US 202263371095 P; 19.05.2023 US 202318320521
(43) Date of publication of application: 14.02.2024
(73) Proprietor: Wright Medical Technology, Inc., Memphis, TN 38117 (US)
(72) Inventor: STEMNISKI, Paul M., Memphis, 38122 (US); KEMPER, Jakob, 2102EW Heemstede (NL); PETERS, Eby, Chanhassen, 55317 (US); HOFFMANN, Ulrich, 79206 Breisach (DE)
(74) Representative: Zimmermann & Partner Patentanwälte mbB

(56) References cited:
- EP-A1- 3 456 274
- WO-A1-2013/018026
- US-A1- 2011 218 542
- US-A1- 2012 130 434
- US-A1- 2020 197 013
- US-A1- 2022 225 868

## Description

### FIELD OF DISCLOSURE

The disclosed system and method relate to improved visualization systems and methods for use in the surgical theater.

### BACKGROUND

Surgical navigation generally provides a surgeon with useful information during surgery. For example, a pose (e.g., at least one of a position and an orientation) of a surgical instrument (e.g., a chisel, drill, trocar, pointer) can be visualized relative to patient-image data. The patient-image data may be two- or three-dimensional image data of a patient acquired using a medical image acquisition technique, such as computed tomography or magnetic resonance imaging. Improved surgical navigation systems, including surgical navigation systems that provide a surgeon with real-time comparison of an intraoperative status compared to a preoperative plan is desirable.
Document US 2011/0218542 A1 relates to systems and instrumentalities for use in total ankle replacement surgery.
Document WO 2013/018026 discloses a positioning device for screws used to fasten an endomedullary nail having a principal direction of extension and presenting at least one hole for the passage of the screws, and which comprises an arm which is connectable to the nail, a bar mounted at a first end of it to the arm and extending substantially parallel to the principal direction of extension of the nail, an alignment head mounted in the vicinity of a second end of the bar opposite the first end, the head being made at least partly of a radio-transparent material, having at least one hole for guiding the fastening screws and being movable relative to the nail through the agency of respective adjustment means in order to bring the at least one guide hole into alignment with the at least one passage hole.

### SUMMARY

The invention is defined in the appended set of claims.

In some aspects, a system includes a targeting cannula. The targeting cannula may extend from a first end to a second end and may define a hole that extends through the targeting cannula from the first end to the second end. The targeting cannula may include at least one visual marker disposed along a length of the targeting cannula. The at least one visual marker may be identifiable in an image obtained by a fluoroscope. The targeting cannula may include a coupling portion adjacent to the second end that may be configured to engage an imaging device.

In some aspects, the coupling portion may include a coupling surface sized and configured to abut a surface of the imaging device.

In some aspects, the coupling surface may be disposed between a first flange and a second flange.

In some aspects, the coupling surface may define at least one hole that is oriented transversely with respect to the hole that extends from the first end to the second end.

In some aspects, the targeting cannula may include at least one ridge.

In some aspects, the at least one visual marker may include a groove.

In some aspects, the at least one visual marker may include a plurality of circumferential grooves.

In some aspects, the system may include a foot-holder assembly and a targeting base configured to be coupled to the foot-holder assembly. The targeting base may be configured to receive at least a portion of the targeting cannula.

In some aspects, the targeting base may include a plate, a first adjustable portion coupled to the plate, and a first actuator configured to facilitate relative movement between the plate and the first adjustable portion.

In some aspects, the targeting base may include a second adjustable portion and a second actuator. The second adjustable portion may be coupled to the plate. The second actuator may be configured to facilitate relative movement between the plate and the second adjustable portion.

In some aspects, the first adjustable portion may be configured to translate the plate in a first direction and the second adjustable portion may be configured to translate the plate in a second direction. The first direction may be different from the second direction.

In some aspects, the targeting base may include an x-y table that may permit linear translation in at least two dimensions.

In some aspects, the plate may define an internal chamber in which a pivoting arrangement is received. The pivoting arrangement may define an opening sized and configured to receive the targeting cannula therein.

In some aspects, the pivoting arrangement may include a slotted ball defining the opening that is sized and configured to receive the targeting cannula.

In some aspects, the pivoting arrangement may include a knob that is configured to rotate relative to plate for locking and unlocking the slotted ball relative to the plate.

In some aspects, the system may include a first imaging device, a display, and a computing device in communication with the first imaging device and the display. The first imaging device may be configured to obtain at least one image of a patient. The computing device may be configured to receive image data corresponding to the at least one image of the patient, identify the at least one visual marker in the image data, and determine a trajectory of the targeting cannula based on a position of the visual marker.

In some aspects, the computing device may be configured to cause a displayed image to be displayed on the display. The displayed image may include at least a portion of the patient with the trajectory of the targeting cannula superimposed.

In some aspects, the computing device may be configured to cause a predetermined trajectory to be superimposed on the displayed image.

In some aspects, the computing device may be configured to cause an identification of an offset between the predetermined trajectory and the trajectory of the targeting cannula to be displayed on the display.

In some aspects, the system may include an adapter and a second imaging device. The adapter may be configured to be coupled to the foot-holder assembly and to support an optical tracking pattern. The second imaging device may be configured to be coupled to the coupling portion of the targeting camera and to obtain an image of the optical tracking pattern.

In some aspects, a reference pattern may be coupled to or supported by the adapter. The reference pattern may include a plurality of radiopaque objects.

In some aspects, a method may include coupling a foot-holder assembly to a patient, inserting a targeting cannula into a targeting base coupled to the foot-holder assembly, obtaining at least one image of an area of interest of the patient, and adjusting an orientation of the targeting cannula based on at least one displayed image. At least a portion of the targeting cannula may be visible in the at least one image obtained of the area of interest of the patient. The displayed image may be based, at least in part, on the at least one image obtained of the area of interest of the patient.

In some aspects, the targeting cannula may extend from a first end to a second end and may define a hole that extends through the targeting cannula from the first end to the second end. The targeting cannula may include at least one visual marker disposed along a length of the targeting cannula. The at least one visual marker may be identifiable in an image obtained by a fluoroscope.

In some aspects, adjusting the orientation of the targeting cannula may include engaging a first actuator coupled to the foot-holder assembly.

In some aspects, engaging the first actuator results in a linear translation of the targeting cannula in a first direction relative to the foot-holder assembly.

In some aspects, adjusting the orientation of the targeting cannula may include engaging a second actuator coupled to the foot-holder assembly.

In some aspects, engaging the second actuator may result in a linear translation of the targeting cannula in a second direction relative to the foot-holder assembly.

In some aspects, the second direction may be different from the first direction.

In some aspects, adjusting the orientation of the targeting cannula may include pivoting the targeting cannula relative to the foot-holder assembly.

In some aspects, adjusting the orientation of the targeting cannula may include pivoting the targeting cannula relative to the foot-holder assembly.

Further disclosed herein is a system that includes a targeting cannula, wherein the targeting cannula extends from a first end to a second end and may define a hole that extends through the targeting cannula from the first end to the second end, wherein the targeting cannula may include at least one visual marker disposed along a length of the targeting cannula, wherein the at least one visual marker may be identifiable in an image obtained by a fluoroscope, and wherein the targeting cannula may include a coupling portion adjacent to the second end that may be configured to engage an imaging device.

### BRIEF DESCRIPTION OF THE DRAWINGS

FIG. 1 illustrates one example of a surgical in accordance with some embodiments;
FIG. 2 illustrates one example of a foot-holder assembly and targeting system in accordance with some embodiments;
FIG. 3 illustrates a top-side view of one example of a targeting base in accordance with some embodiments;

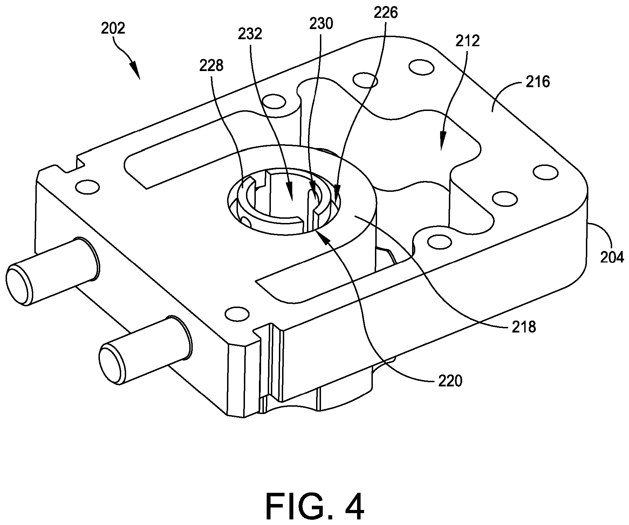

FIG. 4 illustrates a bottom-side view of the targeting base illustrated in FIG. 3 in accordance with some embodiments;

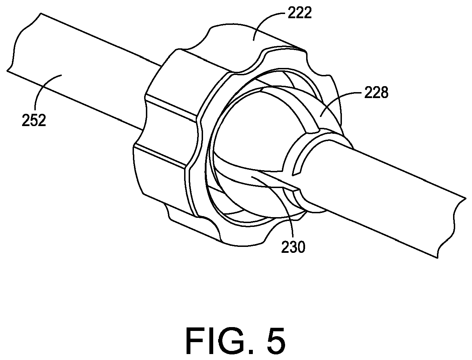

FIG. 5 illustrates a targeting cannula disposed in a slotted ball that may be part of the targeting base illustrated in FIGS. 3 and 4, in accordance with some embodiments;
FIG. 6 illustrates one example of a targeting base configured with adjustable portion in accordance with some embodiments;
FIG. 7 is an isometric view of one example of a targeting cannula in accordance with some embodiments;
FIG. 8 is a side view of the targeting cannula illustrated in FIG. 7 in accordance with some embodiments;
FIG. 9 is a top side view of the targeting cannula illustrated in FIG. 7 in accordance with some embodiments;
FIG. 10 is a flow diagram of one example of a method of performing a surgery with surgical guidance in accordance with some embodiments;
FIG. 11 is a flow diagram of one example of a method for providing surgical guidance in accordance with some embodiments;
FIG. 12A is one example of an image taken in a frontal plane that may be displayed to a user during a surgical procedure showing a divergence between a current trajectory and a preoperatively-determined trajectory, in accordance with some embodiments;
FIG. 12B is one example of an image taken in a sagittal plane that may be displayed to a user during a surgical procedure showing a divergence between a current trajectory and a preoperatively-determined trajectory, in accordance with some embodiments;
FIG. 13A is one example of an image taken in a frontal plane that may be displayed to a user during a surgical procedure showing alignment of a current trajectory and a preoperatively-determined trajectory, in accordance with some embodiments;
FIG. 13B is one example of an image taken in a sagittal plane that may be displayed to a user during a surgical procedure showing alignment of a current trajectory and a preoperatively-determined trajectory, in accordance with some embodiments;
FIG. 14 is another example of a surgical system in accordance with some embodiments;
FIG. 15 illustrates one example of a foot-holder assembly, targeting system, camera, and tracker in accordance with some embodiments;
FIG. 16 is a partially exploded view of a targeting base, targeting cannula, and camera in accordance with some embodiments;
FIG. 17 is an isometric view of the targeting base, targeting cannula, and camera in accordance with some embodiments;
FIG. 18 is a flow diagram of another example of a method of performing a surgery with surgical guidance in accordance with some embodiments;
FIG. 19 is a flow diagram of another example of a method for providing surgical guidance in accordance with some embodiments;
FIG. 20A is one example of an image taken in a frontal plane that may be displayed to a user during a surgical procedure showing a divergence between a current trajectory and a preoperatively-determined trajectory, in accordance with some embodiments;
FIG. 20B is one example of an image taken in a sagittal plane that may be displayed to a user during a surgical procedure showing a divergence between a current trajectory and a preoperatively-determined trajectory, in accordance with some embodiments;
FIG. 21A is one example of an image taken in a frontal plane that may be displayed to a user during a surgical procedure showing alignment of a current trajectory and a preoperatively-determined trajectory, in accordance with some embodiments; and
FIG. 21B is one example of an image taken in a sagittal plane that may be displayed to a user during a surgical procedure showing alignment of a current trajectory and a preoperatively-determined trajectory, in accordance with some embodiments.

### DETAILED DESCRIPTION

This description of the exemplary embodiments is intended to be read in connection with the accompanying drawings, which are to be considered part of the entire written description.

Existing surgical systems, especially those used to perform total ankle replacement ("TAR") surgeries, suffer from drawbacks. For example, the preoperative ("pre-op") plans for surgical systems that include patient-specific components (e.g., jigs, fixtures, locators), tend to be complicated and many aspects cannot be definitively determined preoperatively, such as bone quality and joint laxity and balancing. Further, the lead-time for many of these systems is long making it is difficult to change the pre-op plan.

In addition to the pre-op difficulties and/or drawbacks referenced above, there are additional challenges in the intra-operative and post-operative periods. For example, TAR surgeries are difficult and may be lengthy as the surgical sites may be difficult to visualize, guides may not always fit precisely (e.g., due to residual soft tissue and/or cartilage), and making adjustments may not be easily performed. The lack of visualization may result in extensive use of fluoroscopy in the OR, which has its own set of known drawbacks.

The systems and methods disclosed herein use fluoro-based augmented reality to address these and other problems and drawbacks and issues with conventional surgical systems and methods. The fluoro-based augmented reality may be provided through the use of a combination of two-dimensional ("2D") to three-dimensional ("3D") estimation and/or registration of a surgical site, stereotaxy, and pattern recognition. 2D-to-3D estimation may include acquiring at least one first 2D image, which may be an x-ray image, and at least one second 2D image, which may also be an x-ray image, of a common site (e.g., the surgical site) where the first image is acquired in a first plane and the second image is acquired in a second plane that is disposed at an angle (e.g., an "imaging angle") relative to the first plane. In some embodiments, the imaging angle may be 60 degrees or more, for example. The two 2D images are used to estimate the physical features of the surgical site. For example, the two (or more) 2D images may be used to create a 3D estimation of one or more bones or other physical objects of the patient without the need to obtain one or more 3D images, such as one or more CT scans, of the patient.

2D-to-3D registration may include obtaining one or more 3D images, such as one or more CT scans and/or MRI images. The 3D images may be used to construct a 3D model of the surgical cite, including bone and/or cartilage, as will be understood by one of ordinary skill in the art. One or more 2D images may be obtained and one or more features in the 2D images may be registered to one or more features in the 3D model, as described in U.S. Patent No. 10,667,867, entitled "Methods of Pose Estimation of Three-Dimensional Bone Models in Surgical Planning a Total Ankle Replacement,".

In some embodiments, to facilitate the 2D-to-3D registration, a reference body may be used. The reference body may have a known shape and/or known dimensions to assist in the scaling of the images and the 3D registration. Further, one or more patterns may be provided on the reference body or other object to assist in the aligning the image planes. One example of such a reference body, which may take the form of a plurality of radiopaque spherical beads, is described in U.S. Patent No. 10,105,168, entitled "Stereotactic Computer Assisted Surgery Based on Three-Dimensional Visualization,".

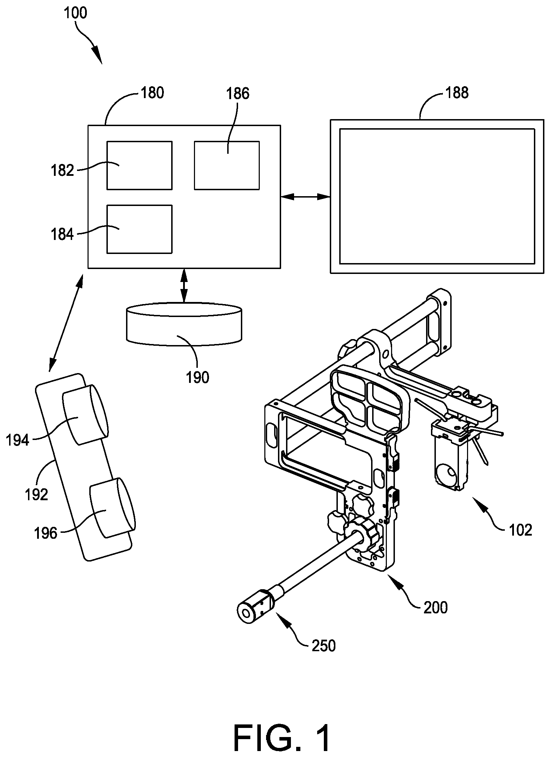

FIG. 1 illustrates one example of a surgical system 100, including a foot-holder assembly 102 having a targeting system 200 in accordance with some embodiments. The surgical system 100 may be used to guide the placement of a tibial stem in connection with a TAR procedure. However, it should be understood that the surgical system 100 may be used in connection with other surgical procedures, including revision procedures and surgical procedures involving other bones and joints.

The surgical system 100 may include a computing device 180, which may have a processor 182, a memory 186, an interface 184, and a display 188. The computing device 180 may be coupled (e.g., by a wireless or a wired connection) to a data storage 190, which may be a database, key-value data store, or other machine-readable storage device. The processor 180 may obtain data from the memory 186 and the data storage 190. The computing device 180 may be configured as a computer terminal located, for example, on a medical cart or may be a handheld tablet. Further, it should be understood that monitor or display 188 may be part of the computing device or may be separate from, but in communication with, computing device 188. One of ordinary skill in the art will understand that the computing device 180 may take other forms.

The computing device 180 may be coupled to an imaging device 192, which may be part of a surgical tracking system. The imaging device 192 may include one or more imaging units 194, 196 (e.g., a C-arm or G-arm) that are configured to obtain images, such as fluoro images, in different planes. For example, if a C-arm with a single imaging unit 194 is used, then the imaging device 192 may obtain a fluoroscopic image in a single plane. If a G-arm with multiple imaging units 194, 196 is used, then the imaging device 192 may obtain fluoroscopic images in multiple planes. The tracking system, using the imaging device 192, may be configured to track a reference body, such as one or more ribs, grooves, fiducial markers, or other objects that may be visible in a fluoroscopic image.

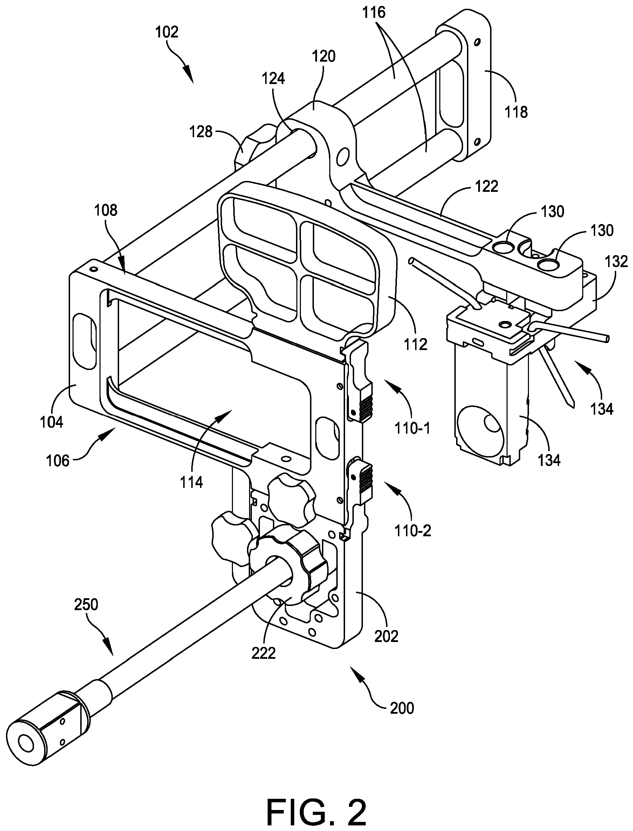

As noted above and as best seen in FIG. 2, surgical system 100 may include a foot-holder assembly 102 and a targeting system 200. In some embodiments, the foot-holder assembly 102 may be similar to the foot-holder assembly 900 disclosed in U.S. Patent No. 8,808,303 and/or foot-holder assembly 1200 disclosed in U.S. Patent No. 8 808,297. The foot-holder assembly 102 may include includes a base plate 104, which may extend from a first side 106 to a second side 108. Each of the first side 106 and the second side 108 may include a respective coupling mechanism 110-1, 110-2 (collectively, "coupling mechanisms 110"). The coupling mechanisms 110 may be configured to couple other components, such as a targeting system 200 and footplate 112, to the foot-holder assembly 102 in a releasable manner as described herein. In some embodiments, the coupling mechanisms 110 may include a spring-loaded detent and a corresponding finger-receiving surface. However, other types of coupling mechanisms may be used such as, for example, slot and groove, dovetail, threads, clamps, and/or tapers, to list only a few possibilities.

Base plate 104 may define a viewing opening 114, which may enable a surgeon to view the bottom of a patient's foot when the foot is secured to foot-holder assembly 102.

One or more rods 116 may extend from base plate 104 in a substantially perpendicular direction with respect to an upper foot-holding surface. Rods 106 may be secured to base plate 104 using screws or through other securing and/or fastening means as will be understood by one skilled in the art. A cap 118 may be coupled to an end of rods 116. The cap 118 may be snap fit onto the end of rods 116, although screws, fasteners, and/or adhesive means may also be used to couple the cap 118 to rods 116.

A mounting member 120 may have an elongate body 122 that may define one or more holes 124 at one end that may be configured to receive rods 116 such that mounting member 120 may be slid along rods 116 in order to position tibial drill guide mount 126 with respect to the base plate 104. In some embodiments, a spring-loaded button or locking knob 128 may be disposed at first end of mounting member 1210 for locking mounting member 120 at a position along rods 116.

One or more holes 130 may be defined at the second end of mounting member 120 and may correspond to holes of a drill guide mounting plate 132 for coupling drill guide mount 126 to the foot-holder assembly 102. The second end of mounting member 120 also may define a slot, which may be sized and configured to receive an internally threaded rod of a pivoting arrangement for securing the drill guide mount 126 to the mounting plate 120.

The targeting system 200, which may include a targeting base 202 and a targeting cannula 250, may be configured to be coupled to the foot-holder assembly 102. The targeting base 202 may include a plate or platform 204 that may be configured to be coupled to the foot-holder assembly 102 in a releasable manner via a coupling mechanism 110. For example, the plate or platform 204 may include one or more notches or slots 206 that may be sized and configured to be engaged by the coupling mechanism 110, which may be a spring-loaded detent. In some embodiments, the platform may also include one or more dowels or pegs, such as pegs 208, extending from a side 210. The pegs 208 may be sized and configured to be received in corresponding holes defined by the foot-holder assembly 102. The combination of the notches 206 and pegs 208 and corresponding spring-loaded detent 110 and holes may be configured to maintain the plate 204 engaged with the foot-holder assembly 102. However, it should be understood that the plate 204 of the targeting base 202 may be coupled to the foot-holder assembly 102 using other means. In some embodiments, the plate 204 of the targeting base 202 may be formed integrally with the foot-holder assembly 102.

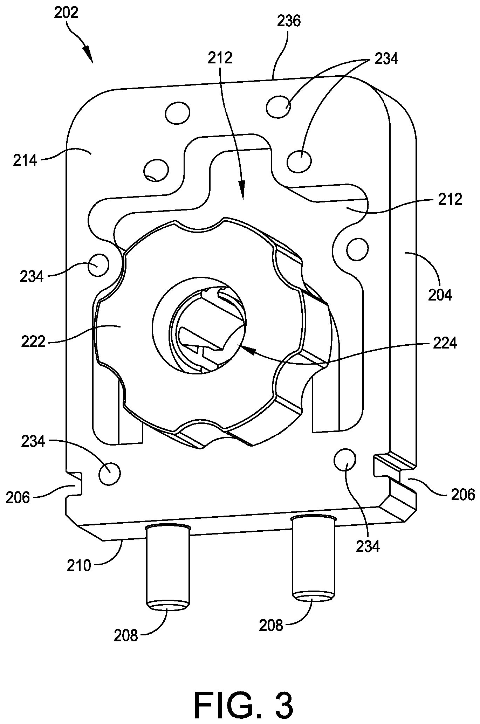

As shown in FIG. 3, the plate 204 may have a square or rectangular shape define one or more openings 212. The one or more openings 212 may extend from a first side 214 (e.g., a surgeon-facing side) through to an opposed second side 216 (e.g., a patient-facing side) and may enable a surgeon or other user to visualize the bottom of the patient's foot while also reducing weight of overall system. The plate 204 may further include a tongue 218 that projects into the opening 212 and may define an aperture 220.

A locking knob 222 may be coupled to the aperture 220 and tongue 218. More particularly, the locking knob 222 may also define a hole 224 that is in communication with an internal chamber 226 in which a pivot ball 228 may be disposed. The pivot ball 228 and knob 222 may be separable from the plate 204 so that the components may be cleaned, as will be understood by one of ordinary skill in the art. In some embodiments, the pivoting ball 228 may include one or more slots 230 and a central opening 232 that extends through the entirety of the ball 228. An internal surface of the locking knob that at least partially defines the internal chamber may be conical or have another geometry that is configured to engage an outer surface of the ball 228 to facilitate clamping or compression of the slotted ball 228 when the locking knob 222 is rotated. As will be understood by one of ordinary skill in the art, locking knob 222 and tongue 218 may have a threaded engagement such that when locking knob 222 is rotated relative to tongue 218, the locking knob 222 translates toward or away from the tongue 218, which compresses or releases the pressure on slotted ball 228.

In some embodiments, a plurality of holes 234 may be defined by the body of the plate 204. The holes 234 may be arranged as pairs of holes that are symmetrical about a centerline that extends from a coupling side 210 of the plate 204 to an opposed second side 236. Holes 234 may be sized and configured to receive a fixation device, such as a pin, k-wire, or other surgical device for coupling the plate to the foot of a patient as will be understood by one of ordinary skill in the art. Further, it should be understood that the holes 234 may be arranged parallel to one another, or the holes 234 may be arranged at other angles.

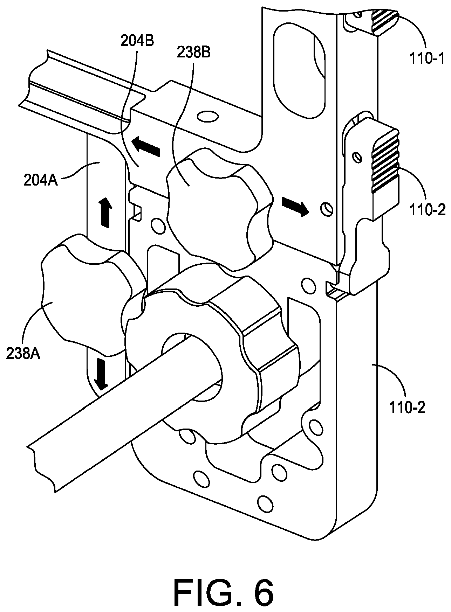

The body of the plate 204 may include one or more adjustable portions configured to allow a surgeon to provide controlled translation intraoperatively. For example, as best seen in FIG. 6, which is a conceptual illustration of the plate 204, the plate 204 may include a first adjustable portion 204A and a second adjustable portion 204B. In some embodiments, the first adjustable portion 204A and the second adjustable portion 204B may be configured to incrementally adjust the position of the plate 204 relative to the base plate 104. A knob or other actuator 238 may be configured to facilitate the translation of the plate 204 relative to the adjustable portions 204A, 204B. For example, rotation of the actuator 238A may result in relative motion between adjustable portion 204A and base plate 104, such as the plate 204 moving in a medial-lateral direction relative to base plate 104. Rotation of the actuator 238B may result in relative motion between adjustable portion 204B and base plate 104 such as the plate 204 moving in an anterior-posterior direction relative to base plate 104. Put another way, the plate 204 may include an x-y table that facilitates the linear adjustment of the location of the central opening 232 relative to the base plate 204 and/or the foot-holder assembly 102.

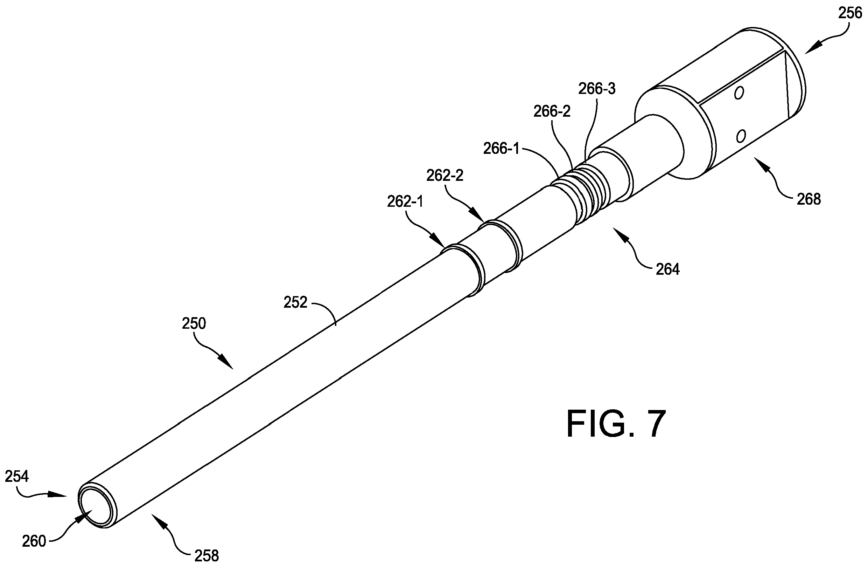

FIGS. 7-9 illustrate one example of a targeting cannula 250 of the targeting system 200, which may be used in connection with the foot-holder assembly 102. The targeting cannula 250 may include an elongate body 252, which may have a generally cylindrical shape, extending from a first end 254 to a second end 256. First end 254 may be a leading end, and second end 256 may be a trailing end or a coupling end. The distal portion 258 of body 252 adjacent to first end 254 may be sized and configured to be received in the central opening 232 defined by the slotted ball 228. In some embodiments, the body 252 of targeting cannula 250 may define a central passageway or hole 260 that extends from the first end 254 to the second end 256.

One or more circumferential ridges 262 may be disposed along a length of the body 252. In some examples, a first ridge 262-1 and second ridge 262-2 are positioned along the length of body 252 such that the ridges 262 are disposed closer to the second end 256 than they are the first end 254. However, it should be understood that the number and placement of the ridges may be varied. Ridges 262 may be sized and configured to provide a stop so that targeting cannula 250 cannot be over-inserted into the central opening 232 defined by the slotted ball 228 of the targeting base 202.

Body 252 of targeting cannula 250 may include visual indicia, queues, or markers 264 that are visible under fluoroscopy. For example, the body 252 of targeting cannula 250 may define one or more circumferential grooves 266 along its length. In the example illustrated in FIGS. 7-9, the body 252 may define a first groove 266-1, a second groove 266-2, and a third groove 266-3. It should be understood that the number of grooves may be varied. As will be understood by one of ordinary skill in the art, the one or more grooves 266 may be configured to provide a visual queue or marker that may be observable in fluoroscopic imaging. Alternative structures to grooves 266 may be provided along the length of body 252, such as fiducial markers, notches, radiopaque objects (e.g., beads, bars, rings), or any other object that may be distinctly identified in one or more fluoroscopic images to provide a visual indicia or marker 264.

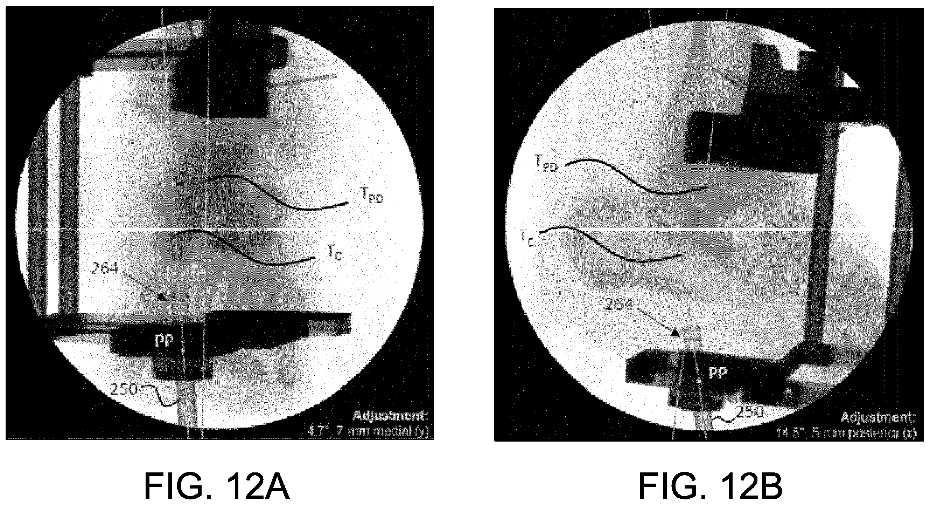

In the example illustrated in FIGS. 7-9, the one or more grooves 266 are positioned between the ridges 262 and the coupling end 256. However, one or more of the one or more grooves 266 may be positioned at other locations along the length of body 252. For example, as shown in FIGS. 12A-13B, one or more grooves 266 may be located adjacent to the leading end 254, e.g., along the distal portion 258. It should be understood that the one or more grooves or other markers 264 may be located along the body of the targeting cannula 250 such that they are visible under fluoroscopy when the targeting cannula 250 is received in or otherwise coupled to the foot-holder assembly 102. One of ordinary skill in the art will understand that the markers, such as the grooves, may be radiopaque such that they are visible under fluoroscopy. Accordingly, all or at least a portion of the targeting cannula 250 may be formed from a radiopaque or radiodense material that is suitable for surgical applications. In some embodiments, some portions of the targeting cannula 250 may be formed from a radiolucent material while other portions may be formed from radiopaque materials to facilitate fluoroscopic visualization.

Trailing end 256 of targeting cannula 250 may include a coupling portion 268. Coupling portion 268 may be sized and configured to be coupled to a camera or other image-obtaining device. In some embodiments, the coupling portion 268 may include at least one camera coupling surface 270 that is disposed between a pair of outwardly extending flanges 272, 274. Flange 272 may be disposed at trailing end 256 and may have generally planar sides 276. Flange 274 may be disposed at an opposite side of the planar section 276 from flange 272. Flange 274 may be shaped as a frustum that has a smaller diameter at its distal end compared to its proximal end.

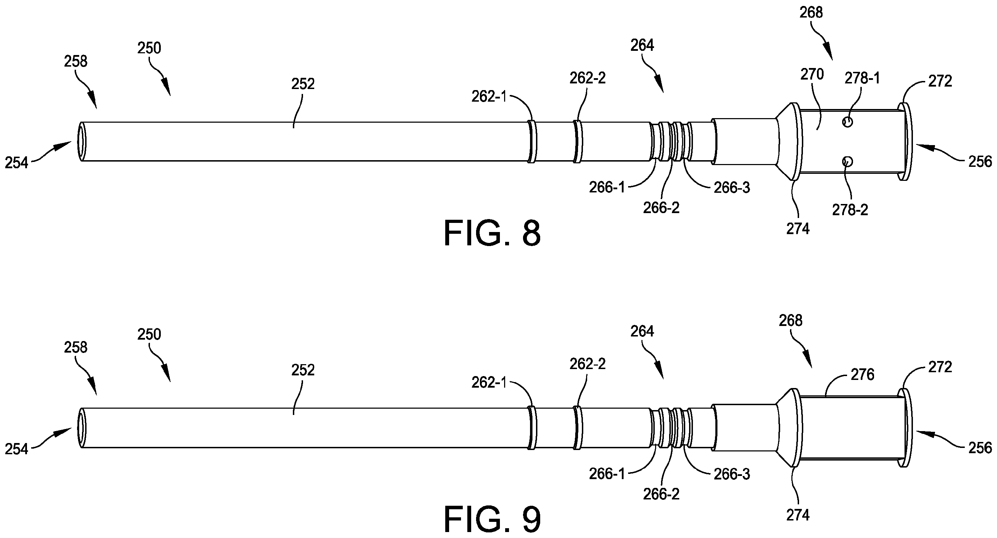

As best seen in FIGS. 8 and 9, the camera coupling surface 270 may be planar, although camera-coupling surface 270 may have other shapes. For example, the shape of camera coupling surface 270 may be complementary to a surface of the camera or image-obtaining device that may be coupled to the camera-coupling surface 270. In some embodiments, the camera coupling surface 270 may define one or more holes 278-1, 278-2 ("holes 278"). Holes 278 may be sized and configured to receive a screw or other fixation device for coupling the camera or image-obtaining device to camera coupling surface 270. In some embodiments, the holes 278 may be oriented transversely with respect to an axis defined hole 260. Holes 278 may be positioned such that the holes 278 do not interfere or communicate with hole 260.

The surgical system 100 may be used to provide a surgeon with a real-time comparison of a trajectory provided by the targeting system 200 (and, specifically, the targeting cannula 250) to a desired trajectory, such as a trajectory in a preoperative plan, during a TAR surgery, such as the surgical procedure disclosed in U.S. Patent No. 7,534,246, Another example of such a TAR surgery is the Prophecy Inbone TAR surgery, which may include obtaining a CT scan from the knee to the foot of a patient. The CT scan may be used to create a preoperative surgical plan, which may include the creation of one or more bone models and/or patient-specific instruments (PSIs), such as a tibial pin alignment guide, a talar pin alignment guide, and a tibial stem alignment guide. Examples of such PSI guides are disclosed in U.S. Patent No. 8,808,303.

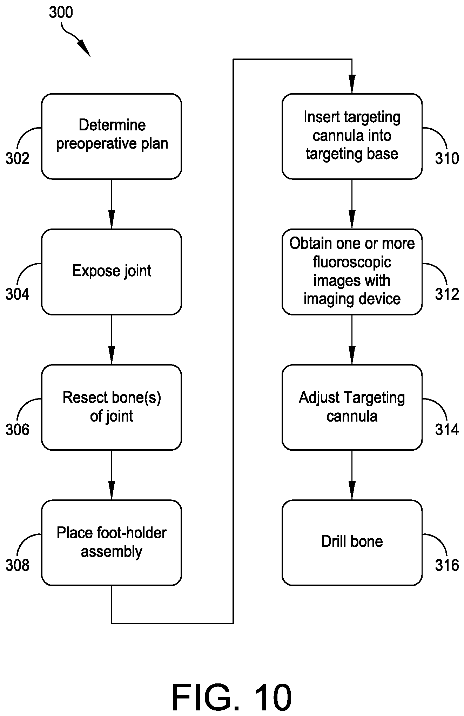

FIG. 10 illustrates one example of a method of performing a surgical procedure. The method 300 shown in FIG. 10 may be performed by one or more individuals, such as by one or more surgeons and/or technicians that may be under the direction of the one or more surgeons.

At block 302, a preoperative plan is determined. As noted above, determining a preoperative plan may include obtaining one or more images, such as CT, MRI, X-ray, or other images, of a patient's anatomy. The surgeon(s) may review the one or more images in determining the preoperative plan. Further, in some embodiments, one or more PSIs may be generated based on the preoperative plan. All or part of the preoperative plan may be stored electronically in a computer-readable storage medium, such as a memory 186 and/or data storage 190.

At block 304, a joint may be exposed. For example, an ankle (i.e., the joint between the tibia and talus) may be exposed.

At block 306, one or more bones of the joint may be resected. In some embodiments, the one or more bones of the joint may be resected using one or more cutting guides. For example, a tibial pin alignment guide, such as the tibial pin alignment guide disclosed in U.S. Patent No. 8,808,303, may be placed on the anterior portion of the tibia. The tibial pin alignment guide may be used to guide the placement of one or more pins into an anterior of the tibial. The tibial pin alignment guide may be removed from its engagement with the tibia leaving the one or more pins in place, and a cutting guide may be slid onto the one or more pins remaining in the tibia. The cutting guide may be used to resect the distal end of the tibia. The one or more pins and the cutting guide may be removed once the distal end of the tibia has been resected.

A talar pin alignment guide, such as the talar pin alignment guide disclosed in U.S. Patent No. 8,808,303, may be placed on the talar dome. The talar pin alignment guide may be used to guide the placement of one or more pins into the talar dome. The talar pin alignment guide may be removed from its engagement with the talar dome leaving the one or more pins in place. A cutting guide, which may be the same or a different cutting guide that was used to resect the tibia, may be slid onto the one or more pins remaining in the talar dome. The cutting guide may be used to resect the talar dome. The one or more pins and the cutting guide may be removed once the talar dome has been resected.

At block 308, the foot-holder assembly 102 may be placed on the patient. In some embodiments, placing the foot-holder assembly 102 on the patient may include inserting a tibial drill guide mount 126 into the resected joint space between the tibia and the talus, including a mounting plate 132 and a drill guide cartridge 134. The mounting plate 132 may be coupled to a PSI (not shown), and the drill guide cartridge 134 may be inserted into the PSI. Pins may be inserted through the mounting plate 132 into the tibia to secure the construct within the resected joint space. The foot-holder assembly 102 may be coupled to the construct by coupling the mounting member 132 of the foot-holder assembly 102 to the mounting plate 132. The targeting base 202 may be coupled to the foot-holder assembly 102 prior to the foot-holder assembly 102 being coupled to the construct or the targeting base 202 may be coupled to the foot-holder assembly after the foot-holder assembly 102 is coupled to the construct. Access to the calcaneus may be obtained before or after the targeting base 2 is coupled to the foot-holder assembly 102, as will be understood by one of ordinary skill in the art.

At block 310, the targeting cannula 250 is inserted into the targeting base 202 coupled to the foot-holder assembly 102. For example, the targeting cannula 250 may be inserted into the central opening 232 defined by the slotted ball 228 of the targeting base 202. In some embodiments, such as embodiments in which leading end of the targeting cannula includes one or more grooves or visual markers 264, the targeting cannula 250 is inserted into the slotted ball 228 such that at least a portion of the leading end 254 of the targeting cannula 250 extends beyond the plate 204 of the targeting base 202 such that the targeting markers 264 are not obscured by the plate 204 and are positioned such that they are visible under fluoroscopy.

At block 312, one or more fluoroscopic images of the ankle of the patient may be obtained using an imaging device 192. For example, a first image may be obtained in the frontal plane, and a second image may be obtained in the sagittal plane. One of ordinary skill in the art will understand that one or more fluoroscopic images may be obtained in other planes.

At block 314, the position and/or orientation of the targeting cannula 250 may be adjusted. The position and/or orientation of the targeting cannula 250 may be adjusted based on one or more images (e.g., targeting guidance) provided to the one or more surgeons and/or other individuals by the surgical system 100, as described in greater detail below.

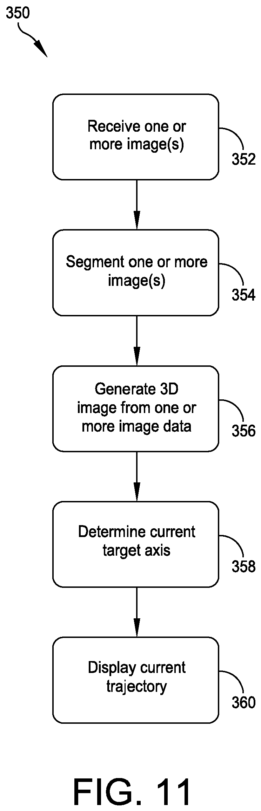

FIG. 11 illustrates one example of a method 350 for providing targeting guidance in accordance with some embodiments. As will be understood by one of ordinary skill in the art, the targeting guidance may be provide by the computing device 180 of the surgical system 100 illustrated in FIG. 1.

At block 352, computing device 180 receives image data corresponding to the one or more images obtained by the imaging device 192 at block 312 of method 300. One of ordinary skill in the art will understand that the image data may be transferred wireless or via a wired connection or coupling from the imaging device 192 to the computing device 180.

At block 354, the computing device 180 may perform one or more segmenting steps to identify the bones of the foot and/or the one or more visual markings 264, such as grooves 266, in the obtained image data.

At block 356, a three-dimensional image may be determined from the image data. Exemplary computer programs for generating three-dimensional images from two-dimensional image data, such as the bi-planar image data, include, but are not limited to, Analyze available from AnalyzeDirect, Inc., Overland Park, Kansas; Insight Toolkit, an open-source software toolkit available from the National Library of Medicine Insight Segmentation and Registration Toolkit ("ITK"), www.itk.org; 3D Slicer, open-source software available from www.slicer.org; Mimics available from Materialise, Ann Arbor, Michigan; and Paraview, available at www.paraview.org, to list only a few examples.

At block 358, the computing device 180 may determine a current target axis defined by the targeting cannula 250 based on the bi-planar image data. For example, the computing device 250 may be able to calculate a central axis of the targeting cannula 250 having detected the grooves 266 or other visual markers 264, as the targeting cannula 250 has dimensions that are known to the computing device. The computing device 180 also may have access to the preoperative plan, which may be stored in data storage 190 or in local storage 186. Using the known dimensions for the targeting device and the data derived from the image data and/or the data obtained from the preoperative plan (e.g., bone models, including dimensions), the computing device 180 may calculate the current trajectory as it relates to the bones in the obtained images.

At block 360, the computing device 180 may display one or more images on the display 188. In some embodiments, the one or more images may include the images obtained using the imaging device 192 with annotations. For example, the annotations may include a line or other annotation showing current a trajectory of the targeting cannula 250. In some embodiments, a desired trajectory, such as the trajectory determined by the preoperative plan, may also be superimposed on the image and displayed. Other information, such as dimensional variance or offsets from the preoperative plan, may also be displayed.

FIGS. 12A-13B illustrate examples of images that may be displayed to a user at block 360 of FIG. 11. More particularly, FIG. 12A is an example image taken in the frontal plane showing the preoperatively determined trajectory T_{PD} and the current trajectory T_{C} of the targeting cannula 250, and FIG. 12B is an example image taken in the sagittal showing the preoperatively determined trajectory T_{PD} and the current trajectory of the targeting cannula Tc.

As shown in FIG. 12A, the current trajectory T_{C} is offset from the preoperatively determined trajectory T_{PD} as is readily apparent from the superimposed lines T_{C}, T_{PD}. The example image shown in FIG. 12A also shows the dimensions of the offset, as determined by computing device 180, which is that the current trajectory is located 7 mm from the preoperatively determined trajectory in the lateral direction (i.e., the current trajectory needs to be moved 7 mm laterally) with an angle between the two trajectories of 4.7°. In some embodiments, the computing device 180 may further identify a location PP about which the target cannula 250 may be pivoted (e.g., pivot point). The pivot point PP may correspond to the center of the slotted ball 228 of the targeting base 202.

In the example image shown in FIG. 12B, the current trajectory T_{C} is shown being offset from the preoperatively determined trajectory T_{PD} by 5 mm in the anterior direction (i.e., the current trajectory needs to be moved in a posterior direction) by 5 mm, and the angle between the two trajectories is 14.5°. Again, the pivot point PP may be displayed in the image, as shown in FIG. 12B.

Referring again to block 314 of method 300 shown in FIG. 10, the surgeon or other user may adjust the trajectory of the targeting cannula 250 based on the guidance provided by the computing device 180. For example, the surgeon or other user may use one or more of the actuators 238 to adjust a translational offset (e.g., an offset in one of the frontal or sagittal planes). For example, the surgeon or user may engage one or both of the actuators 238, such as by pushing, pulling, and/ turning, to cause one or both of the adjustable portions 204A, 204A to move thereby changing the linear position of the targeting cannula 250, which may be received in the slotted ball 228 of the targeting base 202. In some embodiments, the actuators 238 may be coupled to a rack and pinion, worm gear, or other adjustment mechanism that may cause the relative motion. The actuators 238 may be configured so that a predetermined movement of the actuators (e.g., one rotation) corresponds to a predetermined lateral movement of the adjustment portion (e.g., 1 mm). One of ordinary skill in the art will understand that the relationship between movement of the actuators 238A, 238B and the adjustment portions 204A, 204B may be varied and indicia may be provided for signaling to a user the relationship.

Additionally or alternatively, the surgeon or user may adjust an angular position of the targeting cannula 250 by turning the locking knob 222 to loosen the pressure applied by the locking knob 222 onto slotted ball 228. The reduced pressure on slotted ball 228 allows the slotted ball 228 to rotate relative to the locking knob 222 such that the user may adjust the trajectory of the targeting cannula 250. When the desired angular adjustment has been made, the locking knob 222 may be rotated in the opposite direct to increase the force applied to the slotted ball 228 thereby fixing the position of the targeting cannula with respect to the targeting base 202 and foot-holder assembly 102.

The adjustment of the targeting cannula 250 may be performed while the computing device 180 displays real-time images of on the display 188. For example, as the surgeon or other physician or user translates the targeting cannula 250 and/or adjusts an angular orientation of the targeting cannula, the computing device 188 may continuously (e.g., at a predetermined interval, which may be periodic) receive image data from the imaging device 192 and display the relative trajectories (e.g., the trajectory of the targeting guide and the preoperatively determined trajectory) on the display in real-time to assist the surgeon in making the adjustments.

It should be understood that the images may be acquired at a user-defined interval or in response to a user input. For example, to limit the use of fluoroscopy and radiation in the operating theater, a user may determine when or how frequently the one or more fluoroscopic images are obtained. As such, a first set of one or more images may be acquired initially once the targeting cannula 250 has been positioned, and a second set of one or more images may be acquired once the targeting cannula has been repositioned. One of ordinary skill in the art will understand that the computing device 180 may be configured to control the rate at which the images are obtained, and that the interval may be determined and/or adjusted by a user being inputting the desired value into the computing device 180.

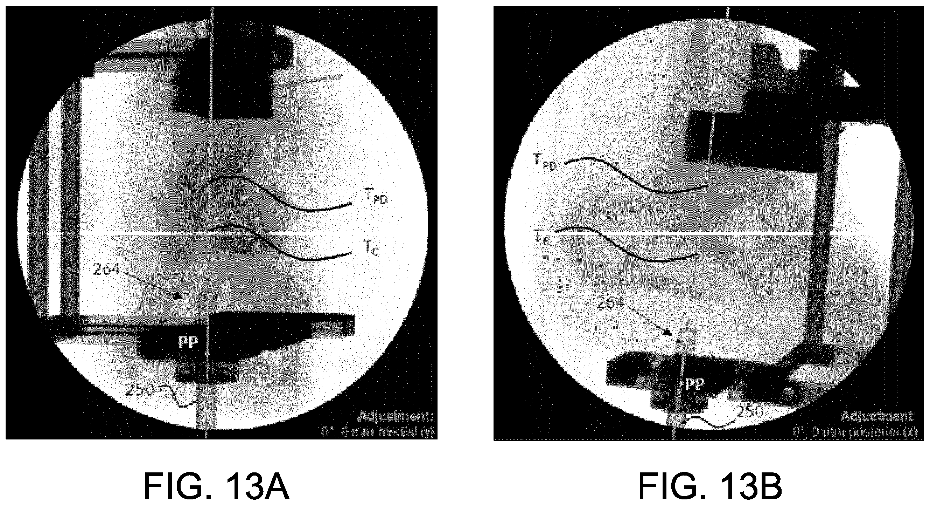

In some embodiments, the adjustment of the targeting cannula 250 and obtaining of the images may be performed in an iterative fashion until proper alignment between the trajectory of the targeting cannula and the preoperative plan has been achieved. FIGS. 13A and 13B are examples of images that may be displayed by the computing device 180 on display 188 showing the alignment of the two trajectories.

Once the trajectories have been aligned, the surgical process may continue. For example, a drill, such as a 6 mm drill, a pin, or other elongate surgical instrument may be inserted into the targeting cannula 250 at block 316. A drill may be used to form a hole through the calcaneus and talus of the patient. The hole may extend into the tibia and serve as a pilot hole for reaming the tibia for receipt of a tibial stem component, such as a multicomponent tibial stem component as described in U.S. Patent No. 7,534,246. One of ordinary skill in the art will understand that the surgical system described herein may be used to perform other surgeries and that the techniques and concepts may be extended to other surgical procedures beyond those involving the ankle.

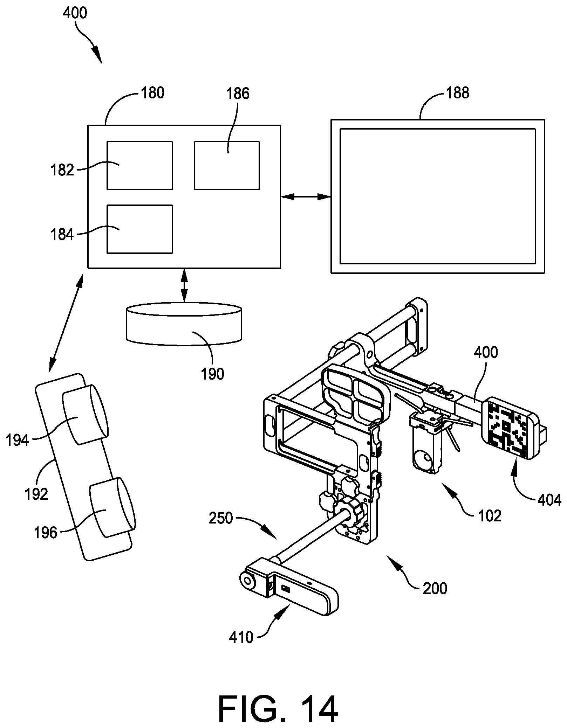

FIG. 14 illustrates another example of a surgical system 400 in accordance with some embodiments. Surgical system 400 may include a computing device 180, foot-holder assembly 102, imaging device 192, and targeting system 200 as described above.

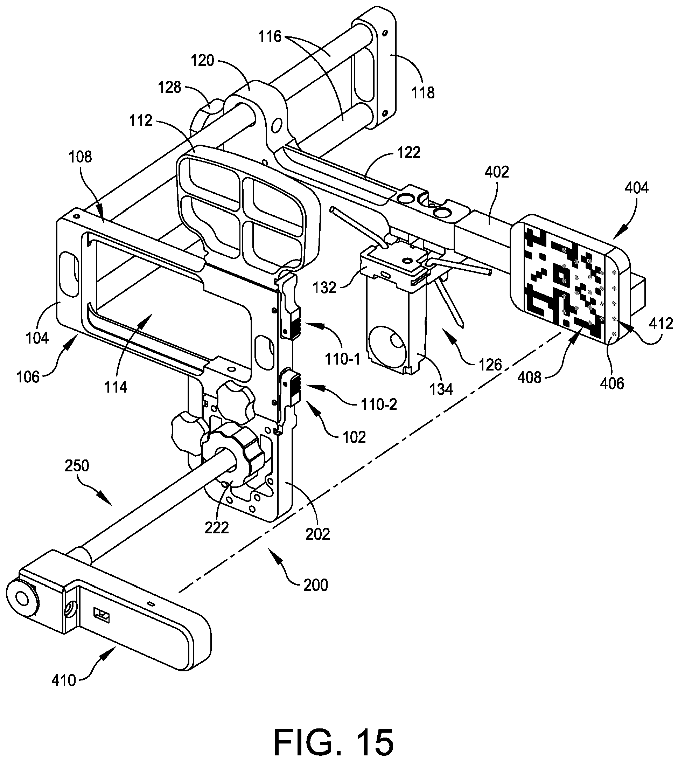

As shown in FIG. 15, an adapter 402 may be coupled to an end of the mounting member 122 of the foot-holder assembly 102. In some embodiments, the adapter 402 may be part of the mounting member 122 (e.g., the adapter and mounting member may be monolithic or fixedly coupled together), although the adapter 402 may be a separate from the mounting member 122. In some embodiments, the adapter 402 may be configured to be coupled to the mounting plate 132 that may be coupled to the mounting member 122 as described above. Here again, the adapter 402 may be part of the mounting plate 132 (e.g., the adapter and the mounting plate may be monolithic or fixedly coupled together) or the adapter 402 may be separate from the mounting plate 402. The adapter 402 may be of any shape or configuration sufficient to support a tracker 404 in a steady position, as will be understood by one of ordinary skill in the art.

Tracker 404 may be coupled to the adapter 402 and may include a flat surface 406 on which an optical tracking pattern 408 may be disposed, as disclosed in U.S. Patent Application Publication No. 2021/0378753, entitled "Technique for Providing User Guidance in Surgical Navigation,". For example, the optical tracking pattern 408 may include a one-, two-, and/or three-dimensional tracking pattern. Examples of optical tracking patterns include, but are not limited to, bar codes and QR codes, to list only a couple of potential examples. In some embodiments, the adapter 402, tracker 404, and/or another component that may be attached or otherwise coupled to the adapter 402 and/or tracker 404 may also include one or more reference bodies 412. For example, the reference bodies 412 may be incorporated in, supported by, or otherwise coupled to the adapter 402, tracker 404, and/or other component such that the reference bodies will be in the field of view of the imaging device 192. The reference bodies 412 may take a variety of forms, including a plurality of radiopaque spherical beads, pins, rings, or other shape arranged in a specific pattern, as described in U.S. Patent No. 10,105,168. The reference bodies 412 may be configured and arranged to communicate position and orientation with a 3D model in a computer system with a visual output for predicted positions with live motion capture. The reference bodies 412 may be provide such that they are visible in both the coronal (e.g., front) and/or sagittal (e.g., side) views for both fluoroscopic and visual imaging. In some embodiments, the adapter 402 and/or tracker 404 may be formed from a radiolucent material and support or incorporate one or more reference bodies 412 that may be formed from a radiopaque material as will be understood by one of ordinary skill in the art.

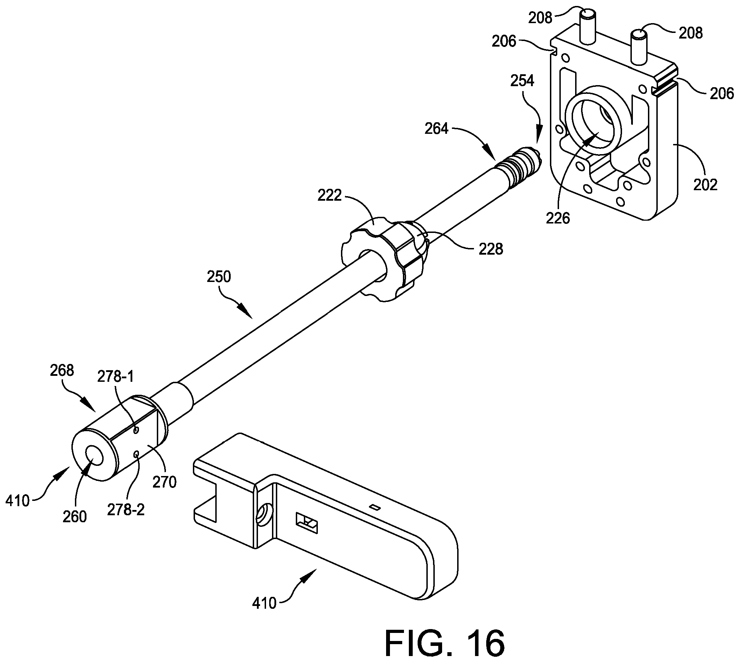

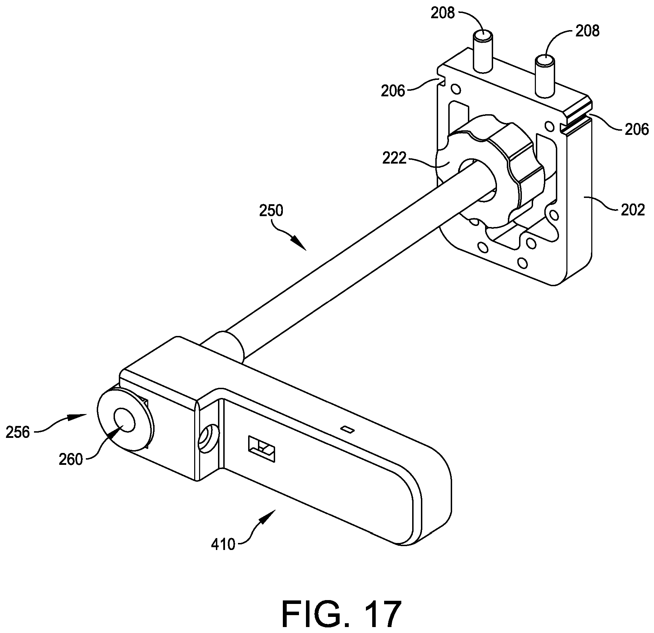

The optical tracking pattern 408 may be configured to be detected in one or more images, as will be understood by one of ordinary skill in the art. In some embodiments, the computing device 108 may be configured to identify the optical tracking pattern 408 in one or more images obtained by a camera 410. As shown in FIGS. 14-17, the camera 410 may be coupled to the coupling portion 268 of the targeting cannula 250. For example, a surface or side of the camera 410 may be placed in contact with the camera coupling surface 270 of the targeting cannula 250. The camera 250 may be coupled to the coupling surface 270 via one or more screws or through other coupling means, such as a snap-fit engagement, detent, or other suitable mechanical or adhesive coupling means as will be understood by one of ordinary skill in the art. Camera 410 may be supported by the coupling portion 268 of the targeting cannula 250 such that a lens of the camera 410 may be aimed or directed at the optical tracking pattern 408.

As noted above, the computing device 180 may be configured to identify the optical tracking pattern 408 in one or more images obtained by the camera 410, which may be communicatively coupled to the computing device 180. Computing device 180 may also be configured to identify the reference bodies 412 in one or more fluoroscopic images obtained by imaging device 192, which may be communicatively coupled to the computing device 180. Computing device 180 also may be able to determine a relative spatial relationship between the targeting cannula 250 and a reference point. In some embodiments, the reference point may be the pivot point PP of the targeting base 202. Determining the relative spatial relationship between the targeting cannula 250 and the reference point PP may enable the computing device to provide real-time information as to the trajectory of the targeting cannula 250 to a surgeon or other user(s) of the surgical system 400.

The computing device 180 also may be configured to determine a relationship between the reference point (or points) and one or more points in a preoperative plan. For example, the computing device 180 may have access to a preoperative plan, which may include one or more bone models and preoperatively determined surgical steps, including a desired trajectory of a drill for drilling a pilot hole from a distal portion of a calcaneus through the tibia and into a tibia. The preoperative plan may be stored in data storage 190, which may be accessible via a network, or in a local storage 186. Using the known dimensions for the targeting system 200 and the data obtained from the image data obtained from the camera 410, the computing device 180 may calculate the current trajectory and compare it to the preoperatively determined trajectory. Computing device may be further configured to display information to one or more users on the display 188 intraoperatively, as described below.

The surgical system 400 illustrated in FIGS. 14-17 may be used to provide a surgeon with a real-time comparison of a trajectory provided by the targeting system 200 (and, specifically, the targeting cannula 250) to a desired trajectory. In some embodiments, the desired trajectory may be a trajectory determined and stored in a preoperative plan, as described above.

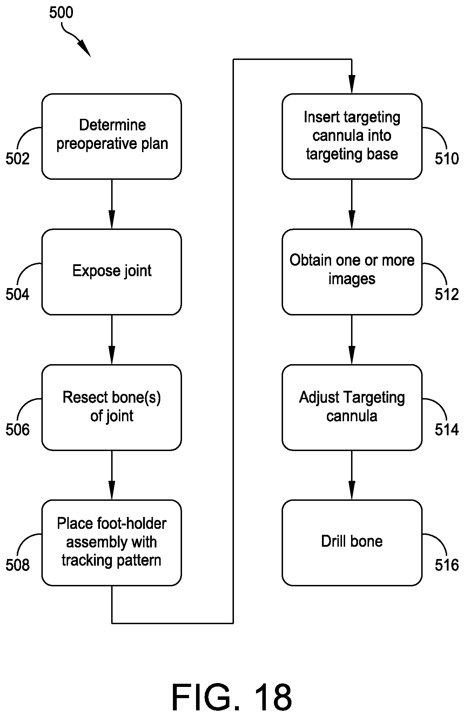

FIG. 18 illustrates one example of a method of performing a surgical procedure. FIG. 18 may be performed by one or more individuals, such as by one or more surgeons and/or technicians that may be under the direction of the one or more surgeons.

At block 502, a preoperative plan is determined. As noted above, determining a preoperative plan may include obtaining one or more images, such as CT, MRI, X-ray, or other images, of a patient's anatomy. The surgeon(s) may review the one or more images in determining the preoperative plan. Further, in some embodiments, one or more PSIs may be generated based on the preoperative plan. All or part of the preoperative plan may be stored electronically in a computer-readable storage medium, such as a memory 186 and/or data storage 190.

At block 504, a joint may be exposed. For example, an ankle (i.e., the joint between the tibia and talus) may be exposed.

At block 506, one or more bones of the joint may be resected. In some embodiments, the one or more bones of the joint may be resected using one or more cutting guides. For example, a tibial pin alignment guide, such as the tibial pin alignment guide disclosed in U.S. Patent No. 8,808,303, may be placed on the anterior portion of the tibia. The tibial pin alignment guide may be used to guide the placement of one or more pins into an anterior of the tibial. The tibial pin alignment guide may be removed from its engagement with the tibia leaving the one or more pins in place, and a cutting guide may be slid onto the one or more pins remaining in the tibia. The cutting guide may be used to resect the distal end of the tibia. The one or more pins and the cutting guide may be removed once the distal end of the tibia has been resected.

A talar pin alignment guide, such as the talar pin alignment guide disclosed in U.S. Patent No. 8,808,303, may be placed on the talar dome. The talar pin alignment guide may be used to guide the placement of one or more pins into the talar dome. The talar pin alignment guide may be removed from its engagement with the talar dome leaving the one or more pins in place. A cutting guide, which may be the same or a different cutting guide that was used to resect the tibia, may be slid onto the one or more pins remaining in the talar dome. The cutting guide may be used to resect the talar dome. The one or more pins and the cutting guide may be removed once the talar dome has been resected.

At block 508, the foot-holder assembly 102, which may be configured with an adapter 402 and tracker 404 having an optical tracking pattern 408, may be placed on the patient. In some embodiments, placing the foot-holder assembly 102 on the patient may include inserting a tibial stem alignment guide 126 into the resected joint space between the tibia and the talus. A mounting plate 132 may be coupled to the tibial stem alignment guide, and a drill guide cartridge 134 may be inserted into the assemblage of a PSI (not shown) and mounting plate 132. Pins may be inserted through the mounting plate 132 and the PSI into the tibia to secure the construct within the resected joint space. The foot-holder assembly 102 may be coupled to the construct by coupling the mounting member 122 of the foot-holder assembly 102 to the mounting plate 122. The targeting base 202 may be coupled to the foot-holder assembly 102 prior to the foot-holder assembly 102 being coupled to the construct or the targeting base 202 may be coupled to the foot-holder assembly 102 after the foot-holder assembly 102 is coupled to the construct. Access to the calcaneus may be obtained before or after the targeting base 202 is coupled to the foot-holder assembly 102, as will be understood by one of ordinary skill in the art.

As described above, an adapter 402 may be coupled to the mounting member 122 of the foot-holder assembly 102 or to the mounting plate 132. A tracker 404 may be coupled to the adapter 402. The tracker 404 may include an optical tracking pattern 408 disposed on all or a part of a surface 406. The optical tracking pattern 408 may be arranged on the tracker 404 such that it may be viewed by a camera 410 that is coupled to the targeting cannula 250. Further, the adapter 402 and/or tracker 404 may include one or more reference bodies 412, which may be positioned in the field of view of the imaging device 192. Although the reference bodies are illustrated as being arranged in a symmetrical or regular pattern (e.g., a grid), it should be understood that the reference bodies may be arranged in an irregular and/or asymmetrical configuration. An irregular or asymmetrical pattern may assist in allowing the system to resolve for symmetries in an image.

At block 510, the targeting cannula 250 is inserted into the targeting base 202 coupled to the foot-holder assembly 102. For example, the targeting cannula 250 may be inserted into the central opening 232 defined by the slotted ball 228 of the targeting base 202. In some embodiments, such as embodiments in which leading end 254 (or distal portion 258) of the targeting cannula 250 includes one or more grooves 266 or visual markers 264, the targeting cannula 250 is inserted into the slotted ball 228 such that at least a portion of the leading end 254 of the targeting cannula extends beyond the plate 404 of the targeting base 402 such that the targeting markers 264 are not obscured by the plate 404 and are positioned such that they are visible under fluoroscopy.

The targeting cannula 250 may be oriented such that the camera 410 coupled to the targeting camera is aimed at the optical tracking pattern 408 disposed on the adapter. The camera 410 may be wired or wirelessly coupled to the computing device 180, and one or more images obtained by the camera 410 may be transmitted to the computing device 180 and displayed on the display 188 to confirm the relative orientation of the camera 410 and optical tracking pattern 408.

At block 512, one or more fluoroscopic images of the ankle of the patient may be obtained using an imaging device 192. For example, a first image may be obtained in the frontal plane, and a second image may be obtained in the sagittal plane. One of ordinary skill in the art will understand that one or more images may be obtained in other planes. The one or more fluoroscopic images may be obtained of the area of interest of the patient and may also include the one or more reference bodies 412 in the field of view as described above. The imaging device 192 may be communicatively coupled to the computing device 180 such that the one or more images obtained by the imaging device 192 may be transferred to the computing device 180.

At block 514, the position and/or orientation of the targeting cannula 250 may be adjusted. The position and/or orientation of the targeting cannula 250 may be adjusted based on one or more images (e.g., targeting guidance) provided to the one or more surgeons and/or other individuals by the surgical system 400, as described in greater detail below.

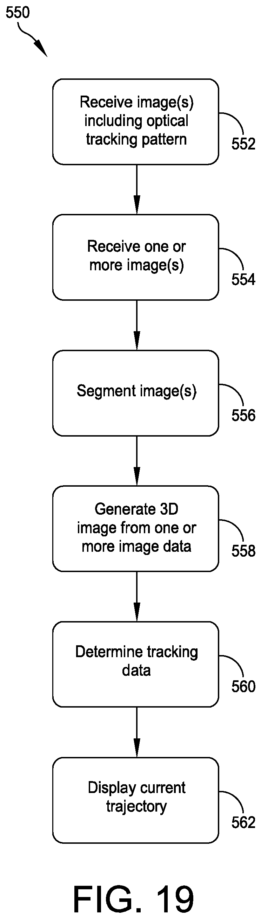

FIG. 19 illustrates one example of a method 550 for providing targeting guidance in accordance with some embodiments. As will be understood by one of ordinary skill in the art, the targeting guidance may be provide by the computing device 180 of the surgical system 400.

At block 552, computing device 180 may receive one or more images from camera 410. The one or more images may include the optical tracking pattern 408. The camera 410 may be coupled to the computing device 180 via a wired or wireless connection as will be understood by one of ordinary skill in the art.

At block 554 computing device 180 may receive image data corresponding to the one or more images obtained by the imaging device 192 at block 512 of method 500. One of ordinary skill in the art will understand that the image data may be transferred wireless or via a wired connection or coupling from the imaging device 192 to the computing device 180.

At block 556, the computing device 180 may perform one or more segmenting steps to identify an object of interest in the one or more images. For example, the computing device 180 may segment one or more images received from camera 410 to identify the optical tracking pattern 408 in the one or more images. Additionally or alternatively, the computing device 180 may segment one or more images received from imaging device 192, which may be a fluoroscope. The fluoroscopic images may be segmented to identify the bones of the foot and/or the one or more visual markings 264 in the obtained image data. The one or more reference bodies 412 may also be identified by the computing device 180.

At block 558, a three-dimensional image may be determined from the image data. Exemplary computer programs for generating three-dimensional images from two-dimensional image data, such as the bi-planar image data, include, but are not limited to, Analyze available from AnalyzeDirect, Inc., Overland Park, Kansas; Insight Toolkit, an open-source software toolkit available from the National Library of Medicine Insight Segmentation and Registration Toolkit ("ITK"), www.itk.org; 3D Slicer, open-source software available from www.slicer.org; Mimics available from Materialise, Ann Arbor, Michigan; and Paraview, available at www.paraview.org, to list only a few examples.

At block 260, the computing device 180 may determine tracking data, including a current target axis defined by the targeting cannula 250 based on the image data and the one or more images that include the optical tracking pattern 408. The tracking data may describe a spatial relationship (e.g., a relative pose) between the targeting cannula 250 and the foot-holder assembly 102. For example, based on the image data, a spatial relationship between the camera 410 and the optical tracking pattern 408 may be determined by localizing at least a part of the optical tracking pattern 408 in the one or more images. The spatial relationship between the imaging device 192 and/or the foot-holder assembly 102 and the targeting cannula 250 may also be determined.

In some embodiments, determining the tracking data may include calculating an aggregate transformation or a chain of transformations as the spatial relationship between the targeting cannula 250 and the foot-holder assembly 102. The aggregate transformation may be determined as a sum of the spatial relationship between the targeting cannula 250 and the camera 410 supported by the foot-holder assembly 102, the spatial relationship between the camera 410 and the optical tracking pattern 408, and the spatial relationship between the optical tracking pattern 408 and the axis defined by the targeting base 402, which may be an axis of the central opening 232 defined by the slotted ball 228. In some embodiments, the aggregate transformation may also depend on a relationship between the foot-holder assembly 102 and the imaging device 192, which may be a C-arm as described above. The relationship between the foot-holder assembly 102 and imaging device 192 may be used in generating the overlay of the trajectory of the targeting cannula 250 on the intra-operative fluoroscopic image(s). This may enable reliably determining the axis defined by the targeting cannula 250 relative to the patient's anatomy.

In some embodiments, the guidance data may describe a plurality of predefined (e.g., preferred) spatial relationships between the targeting cannula 250 and a desired trajectory, which may be the trajectory determined preoperatively by a surgeon. The guidance data may automatically be determined by the one or more processors 182 of computing device 180 or by a different processor(s). The guidance data may be obtained from the memory 186 or from the data storage 190.

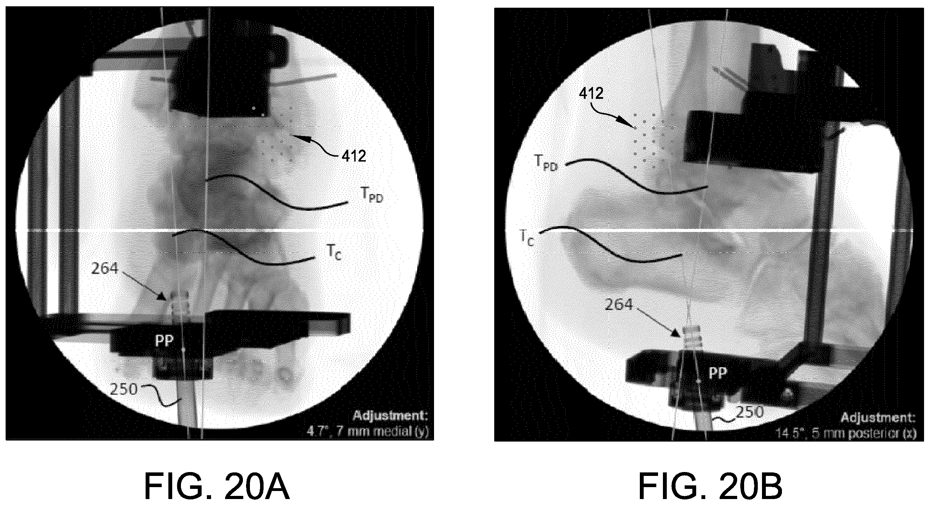

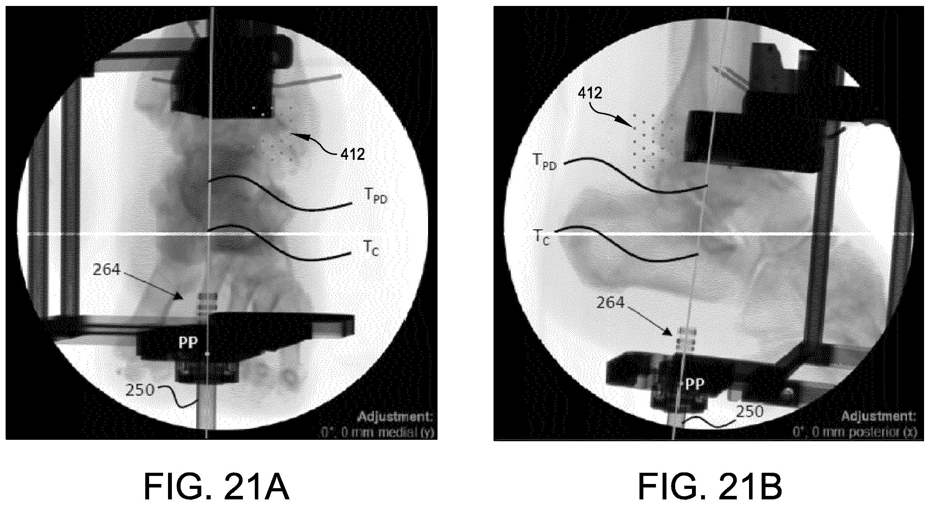

At block 562, the computing device 180 may display one or more images on the display 188. In some embodiments, the one or more images may include the images obtained using the imaging device 192 with annotations. For example, the annotations may include a line or other annotation showing current a trajectory of the targeting cannula 250. In some embodiments, a desired trajectory, such as the trajectory determined by the preoperative plan, may also be superimposed on the image and displayed. Other information, such as dimensional variance or offsets from the preoperative plan, may also be displayed. Examples of such images are shown in FIGS. 12A-13B, which are described above. FIGS. 20A-21B illustrate another set of images that may be displayed. Images 20A-21B are similar to images 12A-13B, but also include the presence of reference bodies 412.

Referring again to block 514 of method 500 shown in FIG. 18, the surgeon or other user may adjust the trajectory of the targeting cannula 250 based on the guidance provided by the computing device 180. For example, the surgeon or other user may use one or more of the actuators 238A, 238B to adjust a translational offset (e.g., an offset in one of the frontal or sagittal planes). For example, the surgeon or user may engage one or both of the actuators 238A, 238B, such as by pushing, pulling, and/ turning, to cause one or both of the adjustable portions 204A, 204B to move thereby changing the linear position of the targeting cannula 250, which may be received in the slotted ball 228 of the targeting base 402. In some embodiments, the actuators 204A, 204B may be coupled to a rack and pinion, worm gear, or other adjustment mechanism that may cause the relative motion. The actuators 204A, 204B may be configured so that a predetermined movement of the actuators (e.g., one rotation) corresponds to a predetermined lateral movement of the adjustment portion (e.g., 1 mm). One of ordinary skill in the art will understand that the relationship between movement of the actuators 204A, 204B and the adjustment portions 204A, 204B may be varied and indicia may be provided for signaling to a user the relationship.

Additionally or alternatively, the surgeon or user may adjust an angular position of the targeting cannula 250 by turning the locking knob 222 to loosen the pressure applied by the locking knob 228 onto slotted ball 228. The reduced pressure on slotted ball 228 allows the slotted ball 228 to rotate relative to the locking knob 222 such that the user may adjust the trajectory of the targeting cannula 250. When the desired angular adjustment has been made, the locking knob 222 may be rotated in the opposite direct to increase the force applied to the slotted ball 228 thereby fixing the position of the targeting cannula 250 with respect to the targeting base 202 and foot-holder assembly 102.

The adjustment of the targeting cannula 250 may be performed while the computing device 180 displays real-time images of on the display 188. For example, as the surgeon or other physician or user translates the targeting cannula 250 and/or adjusts an angular orientation of the targeting cannula, the computing device 180 may continuously (e.g., at a predetermined interval, which may be periodic) receive image data from the imaging device 192 and displays the relative trajectories (e.g., the trajectory of the targeting guide and the preoperatively determined trajectory) on the display in real-time to assist the surgeon in making the adjustments.

It should be understood that the images may be acquired at a user-defined interval or in response to a user input. For example, to limit the use of fluoroscopy and radiation in the operating theater, a user may determine when or how frequently the one or more fluoroscopic images are obtained. As such, a first set of one or more images may be acquired initially once the targeting cannula 250 has been positioned, and a second set of one or more images may be acquired once the targeting cannula has been repositioned. One of ordinary skill in the art will understand that the computing device 180 may be configured to control the rate at which the images are obtained, and that the interval may be determined and/or adjusted by a user being inputting the desired value into the computing device 180. In some embodiments, the adjustment of the targeting cannula 250 and obtaining of the images may be performed in an iterative fashion until proper alignment between the trajectory of the targeting cannula and the preoperative plan has been achieved.

In some embodiments, the one or more fluoroscopic images may be obtained only once. For example, with a camera 410 coupled to the targeting cannula 250, the computing device 180 may be configured to provide real-time guidance without repeatedly capturing one or more fluoroscopic images from the imaging device 192. The computing device 180 may be configured to provide continuous real-time guidance as to the trajectory of the targeting cannula 250 as an overlay on one or more fluoroscopic images previously obtained by the imaging device 192.

Once the trajectories (e.g., trajectory of the targeting cannula and the preoperative trajectory) have been aligned, the surgical process 500 may continue. For example, a drill, such as a 6 mm drill, a pin, or other elongate surgical instrument may be inserted into the targeting cannula 250 at block 516. A drill may be used to form a hole through the calcaneus and talus of the patient. The hole may extend into the tibia and serve as a pilot hole for reaming the tibia for receipt of a tibial stem component, such as a multicomponent tibial stem component as described in U.S. Patent No. 7,534,246. One of ordinary skill in the art will understand that the surgical system described herein may be used to perform other surgeries and that the techniques and concepts may be extended to other surgical procedures beyond those involving the ankle.

One or more of the disclosed methods can be embodied in the form of methods and apparatus for practicing those methods. One or more of the disclosed methods can also be embodied in the form of program code embodied in tangible media, such as floppy diskettes, CD-ROMs, DVD-ROMs, Blu-ray disks, hard drives, or any other machine-readable storage medium, wherein, when the program code is loaded into and executed by a machine, such as a computer, the machine becomes an apparatus for practicing the disclosure. One or more of the disclosed methods may also be embodied in the form of program code, for example, whether stored in a storage medium, loaded into and/or executed by a machine, or transmitted over some transmission medium, such as over electrical wiring or cabling, through fiber optics, or via electromagnetic radiation, wherein, when the program code is loaded into and executed by a machine, such as a computer, the machine becomes an apparatus for practicing the disclosure. When implemented on a general-purpose processor, the program code segments combine with the processor to provide a unique device that operates analogously to specific logic circuits.

## Claims

1. A system (100) for guiding a tibial stem in connection with a total ankle replacement (TAR) procedure, comprising:
a targeting cannula (250) extending from a first end (254) to a second end (256), the targeting cannula (250) defining a hole (260) that extends through the targeting cannula (250) from the first end (254) to the second end (256), the targeting cannula (250) including at least one visual marker (264) disposed along a length of the targeting cannula (250), the at least one visual marker (264) being identifiable in an image obtained by a fluoroscope, the targeting cannula (250) including a coupling portion (268) adjacent to the second end (256), the coupling portion (268) configured to engage an imaging device (192);
a foot-holder assembly (102); and
a targeting base (202) configured to be coupled to the foot-holder assembly (102), the targeting base (202) configured to receive at least a portion of the targeting cannula (250), wherein the targeting base (202) includes:
a plate (204);
a first adjustable portion (204A) coupled to the plate (204); and
a first actuator (238A) configured to facilitate relative movement between the plate (204) and the first adjustable portion (204A),
wherein the targeting base (202) includes:
a second adjustable portion (204B) coupled to the plate (204); and
a second actuator (238B) configured to facilitate relative movement between the plate (204) and the second adjustable portion (204B),
wherein the first adjustable portion (204A) is configured to translate the plate (204) in a first direction, the second adjustable portion (204B) is configured to translate the plate (204) in a second direction, and the first direction is different from the second direction.

2. The system (100) of claim 1, wherein the coupling portion (268) includes a coupling surface (270) that is sized and configured to about a surface of the imaging device (192).

3. The system (100) of claim 2, wherein the coupling surface (270) is disposed between a first flange (272) and a second flange (274), wherein the coupling surface (270) typically defines at least one hole (278) that is oriented transversely with respect to the hole (260) that extends from the first end (254) to the second end (256).

4. The system (100) of any of the preceding claims, wherein the targeting cannula (250) includes at least one ridge (262).

5. The system (100) of claim 4, wherein the at least one visual marker (264) includes a groove (266), or wherein the at least one visual marker (264) includes a plurality of circumferential grooves (266).

6. The system (100) of claim 1, wherein the plate (204) defines an internal chamber in which a pivoting arrangement is received, the pivoting arrangement defining an opening sized and configured to receive the targeting cannula (250) therein.

7. The system (100) of claim 6, wherein the pivoting arrangement includes a slotted ball (228) that defines the opening that is sized and configured to receive the targeting cannula (250), wherein the pivoting arrangement typically includes a knob that is configured to rotate relative to plate (204) for locking and unlocking the slotted ball relative to the plate (204).

8. The system (100) of any of claims 6-7, further comprising:
a first imaging device (192) configured to obtain at least one image of a patient;
a display (188); and
a computing device (180) in communication with the first imaging device (192) and the display (188), the computing device (180) configured to:
receive image data corresponding to the at least one image of the patient;
identify the at least one visual marker (264) in the image data; and
determine a trajectory of the targeting cannula (250) based on a position of the visual marker (264).

9. The system (100) of claim 8, wherein the computing device (180) is configured to cause a displayed image to be displayed on the display (188), the displayed image including at least a portion of the patient with the trajectory of the targeting cannula (250) superimposed.

10. The system (100) of claim 9, wherein the computing device (180) is configured to cause a predetermined trajectory to be superimposed on the displayed image.

11. The system (100) of claim 10, wherein the computing device (180) is configured to cause an identification of an offset between the predetermined trajectory and the trajectory of the targeting cannula (250) to be displayed on the display (188), and the system typically further comprising:
an adapter configured to be coupled to the foot-holder assembly and to support an optical tracking pattern; and
a second imaging device (192) configured to be coupled to the coupling portion of the targeting camera,
wherein the second imaging device (192) is configured obtain an image of the optical tracking pattern.

## Patentansprüche

1. System (100) zum Führen eines Tibiaschafts in Verbindung mit einem Totalendoprothesenverfahren des Sprunggelenks (TAR), umfassend:
eine Zielkanüle (250), die sich von einem ersten Ende (254) zu einem zweiten Ende (256) erstreckt, wobei die Zielkanüle (250) ein Loch (260) definiert, das sich durch die Zielkanüle (250) von dem ersten Ende (254) zu dem zweiten Ende (256) erstreckt, wobei die Zielkanüle (250) mindestens eine visuelle Markierung (264) aufweist, die entlang einer Länge der Zielkanüle (250) eingerichtet ist, wobei die mindestens eine visuelle Markierung (264) in einem durch ein Fluoroskop erhaltenen Bild identifizierbar ist, wobei die Zielkanüle (250) einen Kopplungsabschnitt (268) angrenzend an das zweite Ende (256) aufweist, wobei der Kopplungsabschnitt (268) konfiguriert ist zum Ineingriffnehmen einer Bildgebungsvorrichtung (192);
eine Fußhalterbaugruppe (102); und
eine Zielbasis (202), die konfiguriert ist zum Gekoppeltwerden an die Fußhalterbaugruppe (102), wobei die Zielbasis (202) konfiguriert ist zum Aufnehmen von mindestens einen Abschnitt der Zielkanüle (250), wobei die Zielbasis (202) aufweist:
eine Platte (204);
einen ersten einstellbaren Abschnitt (204A), der an die Platte (204) gekoppelt ist; und
ein erstes Betätigungselement (238A), das konfiguriert ist zum Ermöglichen einer Relativbewegung zwischen der Platte (204) und dem ersten einstellbaren Abschnitt (204A),
wobei die Zielbasis (202) aufweist:
einen zweiten einstellbaren Abschnitt (204B), der an die Platte (204) gekoppelt ist; und
ein zweites Betätigungselement (238B), das konfiguriert ist zum Ermöglichen einer Relativbewegung zwischen der Platte (204) und dem zweiten einstellbaren Abschnitt (204B),
wobei der erste einstellbare Abschnitt (204A) konfiguriert ist zum Verschieben der Platte (204) in eine erste Richtung, der zweite einstellbare Abschnitt (204B) konfiguriert ist zum Verschieben der Platte (204) in eine zweite Richtung, und sich die erste Richtung von der zweiten Richtung unterscheidet.

2. System (100) nach Anspruch 1, wobei der Kopplungsabschnitt (268) eine Kopplungsoberfläche (270) aufweist, die zu etwa einer Oberfläche der Bildgebungsvorrichtung (192) bemessen und konfiguriert ist.

3. System (100) nach Anspruch 2, wobei die Kopplungsoberfläche (270) zwischen einem ersten Flansch (272) und einem zweiten Flansch (274) eingerichtet ist, wobei die Kopplungsoberfläche (270) üblicherweise mindestens ein Loch (278) definiert, das quer in Bezug zu dem Loch (260) orientiert ist, das sich von dem ersten Ende (254) zu dem zweiten Ende (256) erstreckt.

4. System (100) nach einem der vorstehenden Ansprüche, wobei die Zielkanüle (250) mindestens einen Grat (262) aufweist.

5. System (100) nach Anspruch 4, wobei die mindestens eine visuelle Markierung (264) eine Nut (266) aufweist, oder wobei die mindestens eine visuelle Markierung (264) eine Vielzahl von umlaufenden Nuten (266) aufweist.

6. System (100) nach Anspruch 1, wobei die Platte (204) eine interne Kammer definiert, in der eine Schwenkanordnung aufgenommen ist, wobei die Schwenkanordnung eine Öffnung definiert, die bemessen und konfiguriert ist zum Aufnehmen der Zielkanüle (250) darin.

7. System (100) nach Anspruch 6, wobei die Schwenkanordnung eine geschlitzte Kugel (228) aufweist, die die Öffnung definiert, die bemessen und konfiguriert ist zum Aufnehmen der Zielkanüle (250), wobei die Schwenkanordnung üblicherweise einen Knopf aufweist, der konfiguriert ist zum Rotieren relativ zu der Platte (204), zum Verriegeln und Entriegeln der geschlitzten Kugel relativ zu der Platte (204).

8. System (100) nach einem der Ansprüche 6 bis 7, ferner umfassend:
eine erste Bildgebungsvorrichtung (192), die konfiguriert ist zum Erhalten mindestens eines Bilds eines Patienten;
eine Anzeige (188); und
eine Rechenvorrichtung (180) in Kommunikation mit der ersten Bildgebungsvorrichtung (192) und der Anzeige (188), wobei die Rechenvorrichtung (180) konfiguriert ist zum:
Aufnehmen von Bilddaten entsprechend dem mindestens einen Bild des Patienten;
Identifizieren der mindestens einen visuellen Markierung (264) in den Bilddaten; und
Bestimmen einer Trajektorie der Zielkanüle (250) basierend auf einer Position der visuellen Markierung (264).

9. System (100) nach Anspruch 8, wobei die Rechenvorrichtung (180) konfiguriert ist zum Bewirken, dass ein angezeigtes Bild auf der Anzeige (188) angezeigt wird, wobei das angezeigte Bild mindestens einen Abschnitt des Patienten mit der darüber gelegten Trajektorie der Zielkanüle (250) aufweist.

10. System (100) nach Anspruch 9, wobei die Rechenvorrichtung (180) konfiguriert ist zum Bewirken, dass eine vorbestimmte Trajektorie über das angezeigte Bild gelegt wird.

11. System (100) nach Anspruch 10, wobei die Rechenvorrichtung (180) konfiguriert ist zum Bewirken einer Identifikation eines Versatzes zwischen der vorbestimmten Trajektorie und der Trajektorie der Zielkanüle (250) zum Angezeigtwerden auf der Anzeige (188), und das System üblicherweise ferner umfassend:
einen Adapter, der konfiguriert ist zum Gekoppeltwerden an die Fußhalterbaugruppe und zum Stützen eines optischen Verfolgungsmusters; und
eine zweite Bildgebungsvorrichtung (192), die konfiguriert ist zum Gekoppeltwerden an den Kopplungsabschnitt der Zielkamera,
wobei die zweite Bildgebungsvorrichtung (192) konfiguriert ist zum Erhalten eines Bilds des optischen Verfolgungsmusters.

## Revendications

1. Système (100) pour guider une tige tibiale dans le cadre d'une procédure de remplacement total de la cheville (TAR), comprenant :
une canule de guidage (250) s'étendant d'une première extrémité (254) à une deuxième extrémité (256), la canule de guidage (250) définissant un trou (260) qui s'étend à travers la canule de guidage (250) de la première extrémité (254) à la deuxième extrémité (256), la canule de guidage (250) comportant au moins un marqueur visuel (264) disposé sur une longueur de la canule de guidage (250), l'au moins un marqueur visuel (264) étant identifiable dans une image obtenue par un fluoroscope, la canule de guidage (250) comportant une partie de couplage (268) adjacente à la deuxième extrémité (256), la partie de couplage (268) étant configurée pour se mettre en prise avec un dispositif d'imagerie (192) ;
un ensemble de maintien de pied (102) ; et
une base de ciblage (202) configurée pour se coupler à l'ensemble de maintien de pied (102), la base de ciblage (202) étant configurée pour recevoir au moins une partie de la canule de guidage (250), dans lequel la base de ciblage (202) comporte :
une plaque (204) ;
une première partie réglable (204A) couplée à la plaque (204) ; et
une premier actionneur (238A) configuré pour faciliter un déplacement relatif entre la plaque (204) et la première partie réglable (204A),
dans lequel la base de ciblage (202) comporte :
une deuxième partie réglable (204B) couplée à la plaque (204) ; et
un deuxième actionneur (238B) configuré pour faciliter un déplacement relatif entre la plaque (204) et la deuxième partie réglable (204B),
dans lequel la première partie réglable (204A) est configurée pour déplacer par translation la plaque (204) dans une première direction, la deuxième partie réglable (204B) est configurée pour déplacer par translation la plaque (204) dans une deuxième direction, et la première direction est différente de la deuxième direction.

2. Système (100) selon la revendication 1, dans lequel la partie de couplage (268) comporte une surface de couplage (270) qui est dimensionnée et configurée pour environ une surface du dispositif d'imagerie (192).

3. Système (100) selon la revendication 2, dans lequel la surface de couplage (270) est disposée entre une première bride (272) et une deuxième bride (274), dans lequel la surface de couplage (270) définit généralement au moins un trou (278) qui est orienté transversalement par rapport au trou (260) qui s'étend de la première extrémité (254) à la deuxième extrémité (256).

4. Système (100) selon l'une des revendications précédentes, dans lequel la canule de guidage (250) comporte au moins une arête (262).

5. Système (100) selon la revendication 4, dans lequel l'au moins un marqueur visuel (264) comporte une rainure (266), ou dans lequel l'au moins un marqueur visuel (264) comporte une pluralité de rainures (266) circonférentielles.

6. Système (100) selon la revendication 1, dans lequel la plaque (204) définit une chambre interne dans laquelle un agencement pivotant est reçu, l'agencement pivotant définissant une ouverture dimensionnée et configurée pour recevoir la canule de guidage (250) à l'intérieur.

7. Système (100) selon la revendication 6, dans lequel l'agencement pivotant comporte une bille fendue (228) qui définit l'ouverture qui est dimensionnée et configurée pour recevoir la canule de guidage (250), dans lequel l'agencement pivotant comporte généralement un bouton qui est configuré pour tourner par rapport à la plaque (204) pour verrouiller et déverrouiller la bille fendue par rapport à la plaque (204).

8. Système (100) selon l'une des revendications 6 à 7, comprenant en outre :
un premier dispositif d'imagerie (192) configuré pour obtenir au moins une image d'un patient ;
un affichage (188) ; et
un dispositif informatique (180) en communication avec le premier dispositif d'imagerie (192) et l'affichage (188), le dispositif informatique (180) étant configuré pour :
recevoir des données d'image correspondant à l'au moins une image du patient ;
identifier l'au moins un marqueur visuel (264) dans les données d'image ; et
déterminer une trajectoire de la canule de guidage (250) sur la base d'une position du marqueur visuel (264).

9. Système (100) selon la revendication 8, dans lequel le dispositif informatique (180) est configuré pour provoquer l'affichage d'une image affichée sur l'affichage (188), l'image affichée comportant au moins une partie du patient sur laquelle est superposée la trajectoire de la canule de guidage (250).

10. Système (100) selon la revendication 9, dans lequel le dispositif informatique (180) est configuré pour provoquer la superposition d'une trajectoire prédéterminée sur l'image affichée.

11. Système (100) selon la revendication 10, dans lequel le dispositif informatique (180) est configuré pour provoquer l'affichage d'une identification d'un décalage entre la trajectoire prédéterminée et la trajectoire de la canule de guidage (250) sur l'affichage (188), et le système comprenant en outre généralement :
un adaptateur configuré pour être couplé à l'ensemble de maintien de pied et pour supporter un motif de suivi optique ; et
un deuxième dispositif d'imagerie (192) configuré pour être couplé à la partie de couplage de la caméra de ciblage,
dans lequel le deuxième dispositif d'imagerie (192) est configuré pour obtenir une image du motif de suivi optique.
